# EUROPÄISCHE PATENTANMELDUNG

(11) **EP 0 575 754 A1**
(43) Veröffentlichungstag der Anmeldung: **29.12.1993**
(21) Anmeldenummer: 93108239.0
(22) Anmeldetag: 21.05.1993
(51) Int. Cl.: C07D 413/04, A61K 31/41

(54) **Pyridyl-1,2,5-oxadiazol-carbonamid-2-oxide zur Behandlung von NO-abhängigen dysfunktionen, wie Angina Pectoris und Erektionsstörungen**

(30) Priorität: 05.06.1992 DE 4218582
(71) Anmelder: HOECHST AKTIENGESELLSCHAFT, D-65926 Frankfurt am Main (DE)
(72) Erfinder: Schönafinger, Karl, Dr., W-8755 Alzenau (DE); Bohn, Helmut, Dr., W-6369 Schoneck 1 (DE)

(57) **Zusammenfassung**

Die vorliegende Erfindung betrifft Pyridyl-1,2,5-oxadiazol-carbonamid-2-oxide der allgemeinen Formel I 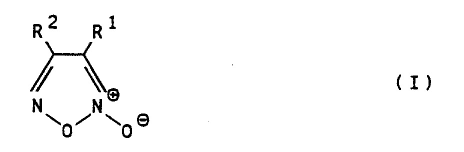
worin einer der Reste R¹ und R² für Pyridyl und der andere für 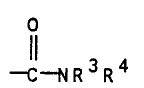
steht und R³ und R⁴ wie in Anspruch 1 angegeben definiert sind, Verfahren zu ihrer Herstellung und ihre Verwendung.

## Beschreibung

Die vorliegende Erfindung betrifft Pyridyl-1,2,5-oxadiazol-carbonamid-2-oxide, Verfahren zu ihrer Herstellung und ihre Verwendung.

Es sind bereits verschiedene 1,2,5-Oxadiazol-carbonamid-2-oxide bekannt und beispielsweise in der EP-B 054 872 und EP-B 054 873 beschrieben. Pyridyl-substituierte Verbindungen dieser Klasse sind bisher allerdings nicht beschrieben.

Die vorliegende Erfindung betrifft Pyridyl-1,2,5-oxadiazol-carbonamid-2-oxide der allgemeinen Formel I 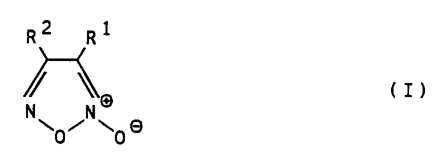
worin einer der Reste R¹ und R² für Pyridyl und der andere für 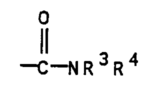
steht,
wobei
R³ und R⁴ unabhängig voneinander Wasserstoff, (C₁-C₆)-Alkyl, (C₅-C₇)-Cycloalkyl, -(CH₂)ₙ-NR⁵R⁶, -(CH₂)ₙ-OR⁵, -(CH₂)ₘ-COOR⁵, -CH(Alk)-COOR⁵, -(CH₂)ₘ-CONR⁵R⁶, -CH(Alk)-CONR⁵R⁶, 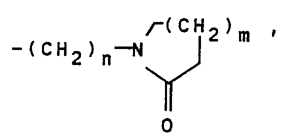
-(CH₂)ₘ-Aryl oder -(CH₂)ₘ-Heteroaryl bedeuten oder R³ und R⁴ zusammen mit dem diese bindenden Stickstoffatom einen Heterocyclus bilden;
R⁵ und R⁶ unabhängig voneinander Wasserstoff, (C₁-C₆)-Alkyl, (C₅-C₇)-Cycloalkyl, Benzyl, Phenethyl bedeuten; Alk (C₁-C₆)-Alkyl bedeutet, sowie
n für 2, 3 oder 4 und
m für 0, 1, 2 oder 3 stehen,
sowie deren pharmakologisch annehmbare Säureadditionsverbindungen.

Die für R³, R⁴, R⁵, R⁶ oder Alk stehenden (C₁-C₆)-Alkyl-gruppen können geradkettig oder verzweigt sein. Beispiele sind Methyl, Ethyl, n-Propyl, i-Propyl, n-Butyl, i-Butyl, sek.-Butyl, tert.-Butyl oder Hexyl.

Für R³, R⁴, R⁵ oder R⁶ stehendes (C₅-C₇)-Cycloalkyl bedeutet bevorzugt Cyclopentyl oder Cyclohexyl.

In der für R³ oder R⁴ stehenden -(CH₂)ₘ-Aryl-Gruppe ist Aryl bevorzugt 6- bis 14-gliedrig. Bevorzugte Reste -(CH₂)ₘ-Aryl sind Phenyl, Benzyl und Phenylethyl.

In der für R³ oder R⁴ stehenden -(CH₂)ₘ-Heteroaryl-Gruppe ist Heteroaryl bevorzugt 5- bis 7-gliedrig und leitet sich beispielsweise von Pyrrol, Pyrrolidin, Imidazol, Pyridin, Piperidin, Morpholin oder Piperazin ab.

Die Aryl- und Heteroarylgruppen können gegebenenfalls auch ein- oder mehrfach substituiert sein. Geeignete Substituenten sind beispielsweise (C₁-C₄)-Alkyl, (C₁-C₄)-Alkoxy, Amino, (C₁-C₄)-Alkylamino, Di-(C₁-C₄)-alkylamino, (C₁-C₆)-Alkanoylamino, Halogen, vorzugsweise Fluor, Chlor oder Brom, Hydroxy, Nitro oder Cyano.

Ein aus R³, R⁴ und dem diese bindenden Stickstoffatom gebildeter Heterocyclus ist beispielsweise Pyrrolidin, Piperidin, Morpholin oder Piperazin, wobei am Piperazin das zweite Stickstoffatom auch durch einen Rest R⁵ substituiert sein kann.

R³ und R⁴ bedeuten unabhängig voneinander bevorzugt Wasserstoff oder (C₁-C₆)-Alkyl. Es ist darüberhinaus bevorzugt, wenn R³ -(CH₂)ₙN((C₁-C₆)-Alkyl)₂ und R⁴ Wasserstoff bedeuten. Besonders bevorzugt bedeuten R³ Methyl und R⁴ Wasserstoff. Der für R¹ oder R² stehende Pyridylrest kann über die 2-, 3- oder 4-Position gebunden sein. Pyrid-3-yl ist bevorzugt.

Die Verbindungen der allgemeinen Formel I können beispielsweise dadurch hergestellt werden, daß eine Verbindung der allgemeinen Formel II 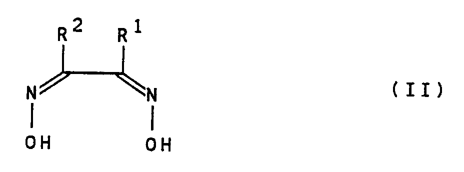
worin R¹ und R² wie oben angegeben definiert sind, oxidiert wird.

Als Oxidationsmittel können dabei herkömmliche Reagentien wie zum Beispiel Halogene, Alkalihypochlorite, Blei(IV)-acetat, Eisen-III-salze wie beispielsweise rotes Blutlaugensalz oder nitrose Gase, wie beispielsweise N₂O₄ eingesetzt werden. Die Umsetzung wird bevorzugt in einem Lösungsmittel, wie beispielsweise Wasser, einem Alkohol, einem Ether, Essigester, Methylenchlorid, Cyclohexan, DMF, DMSO, Benzol, Toluol oder Chlorbenzol bei Temperaturen von -10^{o}C bis 50^{o}C, vorzugsweise von -5^{o}C bis 25^{o}C ausgeführt.

Bei der genannten Oxidation fallen in der Regel die Verbindungen der allgemeinen Formel I in Form von Isomerengemischen an. Diese lassen sich aber durch bekannte Methoden wie Umkristallisieren oder chromatographische Methoden, insbesondere Säulenchromatographie, trennen. Isomerengemische werden auch erhalten, wenn ein reines Isomeres in Substanz oder in einem inerten Lösungsmittel gelöst auf Temperaturen von 50 bis 200^{o}C erhitzt oder bei 0 bis 50^{o}C photolysiert wird. Durch Trennung des so erhaltenen Gemisches ist es somit möglich, ein Isomeres in das andere umzuwandeln.

Die Verbindungen der allgemeinen Formel II können hergestellt werden durch Umsetzung von Verbindungen der allgemeinen Formel III 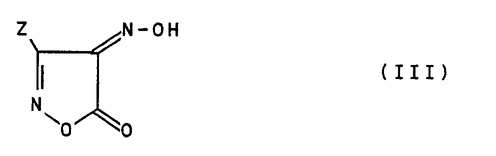
mit einem Amin HNR³R⁴, wobei Z Pyridyl bedeutet und R³ und R⁴ wie oben angegeben definiert sind. Die Umsetzung wird bevorzugt in einem inerten Lösungsmittel bei Temperaturen von 0 bis 50^{o}C ausgeführt.

Die Herstellung der Verbindung der Formel III ist beschrieben in Ann.Chim.(Rome) (1968), 58(2), 189-199, und in Ann.Chim.(Rome) (1959), 49, 2083-2088.

Ein alternatives Verfahren zur Herstellung von erfindungsgemäßen Verbindungen der allgemeinen Formel I besteht darin, eine Verbindung der allgemeinen Formel IV 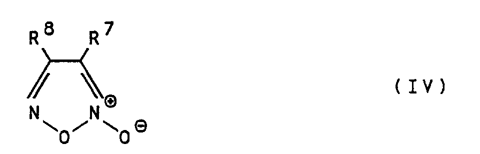
worin einer der Reste R⁷ und R⁸ für Pyridyl und der andere für eine reaktive Säuregruppe, wie zum Beispiel 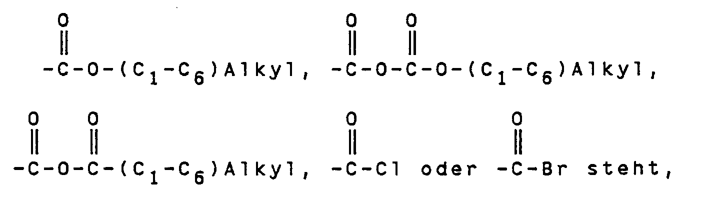
mit einem Amin HNR³R⁴, worin R³ und R⁴ wie oben angegeben definiert sind, umzusetzen.

Vorteilhafterweise wird die Reaktion in Gegenwart einer Base ausgeführt, die die entstehenden Säuren neutralisiert. Bevorzugte Basen sind Alkalicarbonate, wie Natrium- oder Kaliumhydrogencarbonat oder Natrium- oder Kaliumcarbonat, Alkalihydroxide, wie Natrium-, Kalium- oder Lithiumhydroxid, Alkalialkoholate, wie Natrium- oder Kaliummethylat, Natrium- oder Kaliumethylat oder Kaliumtert.-butylat, Alkalihydride, wie Natrium-oder Kaliumhydrid, Alkaliamide, wie Natriumamid oder Lithiumdiisopropylamid oder organische Basen wie Pyridin oder Triethylamin. Diese Basen werden bevorzugt in molaren Mengen eingesetzt. Als Lösungsmittel kommen beispielsweise Ether, THF, Alkohole, Toluol, DMF und DMSO in Frage. Die Temperaturen liegen bei 0 bis 100^{o}C, bevorzugt 0 bis 50^{o}C.

Gegebenenfalls können die nach einem der obenstehenden Verfahren hergestellten erfindungsgemäßen Verbindungen der allgemeinen Formel I durch Modifizierung der Substituenten in weitere erfindungsgemäße Verbindungen der allgemeinen Formel I umgewandelt werden.

Beispielsweise kann die Seitenkette -CO-NH-C(CH₂)ₘCOOR⁵ durch Umsetzung mit einem Amin HNR⁵R⁶ in die Seitenkette -CO-NH-(CH₂)ₘCONR⁵R⁶ umgewandelt weren. Analoges ist mit der Seitenkette -CO-NH-CH(Alk)-COOR⁵ möglich.

Nach den oben angegebenen Verfahren lassen sich beispielsweise erfindungsgemäße Verbindungen der allgemeinen Formel Ia 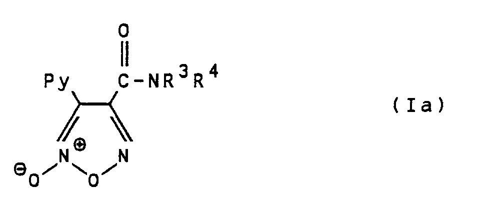
sowie der allgemeinen Formel Ib 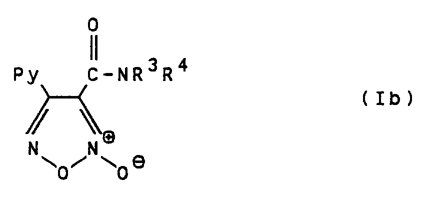
herstellen, worin Py für einen 2-, 3- oder 4-Pyridylrest und -NR³R⁴ jeweils steht für:
-NH₂; -NHCH₃; -NHCH₂CH₃; -NH(CH₂)₂CH₃; -NH(CH₂)₃CH₃; -NH(CH₂)₄CH₃; -NH(CH₂)₅CH₃; -NHcycloC₆H₁₁; -NHcycloC₅H₉; -NH(CH₂)₂N(CH₃)₂; -NH-(CH₂)₂N(CH₂CH₃)₂; -NH(CH₂)₂N(CH₂CH₂CH₂CH₃)₂; -NH-(CH₂)₂N(CH(CH₃)₂)₂; -NH(CH₂)₂NHCH(CH₃)₂; -NH(CH₂)₂NHcycloC₆H₁₁; -NH(CH₂)₃N(CH₂CH₃)₂; 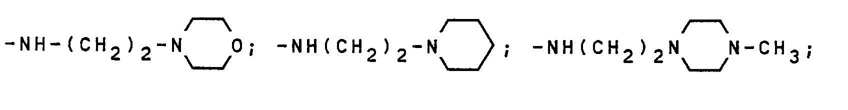
-NHCH₂CH(CH₃)₂; -NH(CH₂)₃N(CH₃)₂; 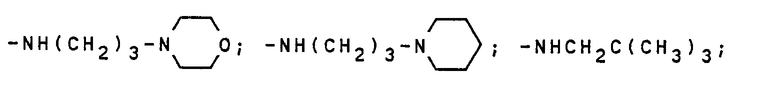
-NH(CH₂)₄N(CH₃)₂; -NH(CH₂)₃NHcycloC₆H₁₁; -NH(CH₂)₃NHC(CH₃)₃; -NH(CH₂)₂OCH₃; -NH(CH₂)₂OCH₂CH₃; -NH(CH₂)₂O(OH₂)CH₃; -NH(CH₂)₂OCH(CH₃)₂; -NH(CH₂)₃OCH₃; -NH(CH₂)₄OCH₃; -NH(CH₂)₃OCH₂CH₃; -NH(CH₂)₃O(CH₂)₃CH₃; -NH(CH₂)₂OH; -NH(CH₂)₃OH; -NH(CH₂)₂OcycloC₅H₉; -NH(CH₂)₂Phenyl; -NHCH₂Phenyl; -NH(CH₂)₃Phenyl; -NH(CH₂)₂(3,4-Di-OCH₃-Phenyl); -NHCH₂(4-OCH₃-Phenyl); -NHCH₂COOCH₃; -NHCH₂COOCH; -NHCH₂COOCH₂CH₃; -NHCH₂CONH₂; -NHCH₂CONHCH₃; -NHCH₂CON(CH₂CH₃)₂; -NH(CH₂)₂COOH; -NH(CH₂)₂COOCH(CH₃)₂: -NH(CH₂)₂CONHCH₂CH₃; -NH(CH₂)₃COOH; -NH(CH₂)₃CONH(CH₂)₃CH₃; -NH(CH₂)₃CONH₂; -NH(CH₂)₃COOCH₂CH₃; -NHCH₂(3-Pyridyl); -NH(CH₂)₂(4-Pyridyl); -NH(CH₂)₂(4-Imidazolyl); -NHCH₂(2-Pyridyl); -NHCH(CH₃)COOH; -NHCH(CH(CH₃)₂)CONH₂; -NHCH(CH₂CH(CH₃)₂)CON(CH₃)₂; -NH(CH₂)₂(2-Oxo-pyrrolidin-1-yl) oder -NH(CH₂)₃(2-Oxo-pyrrolidin-1-yl).

Erfindungsgemäße Verbindungen der allgemeinen Formel I, die eine basische Gruppe enthalten, können mit anorganischen oder organischen Säuren Salze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Die Verbindungen der allgemeinen Formel I und ihre pharmakologisch annehmbaren Säureadditionssalze besitzen wertvolle pharmakologische Eigenschaften. Am Modell der Kalium-depolarisierten Pulmonalarterie des Meerschweinchen führen sie in niedrigen Konzentrationen zu einer lang anhaltenden Relaxation. Diese Wirkung kann mit Oxyhämoglobin inhibiert werden, was auf einen NO-mediierten Mechanismus deutet. Stickstoffmonoxid führt als Aktivator der Guanylatcyclase zu einer Erhöhung von cyclischem Guanosinmonophosphat, welches im glatten Muskel eine Relaxation und in den Blutplättchen antiadhäsive und antiaggregatorische Wirkungen verursacht. Stickstoffmonoxid ist außerdem auch entscheidend beteiligt bei Lernvorgängen, bei der Regulation der Nierenfunktion, bei der Immunabwehr, beim septischen Schock und bei erektilen Dysfunktionen. Die erfindungsgemäßen Verbindungen können somit bei den genannten Indikationen eingesetzt werden.

Vor allem aber haben sich NO-Donoren zur Behandlung und Prophylaxe von angina pectoris bewährt.

Die Verbindungen der allgemeinen Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der allgemeinen Formel I, ihre pharmakologisch annehmbaren Säureadditionsssalze und pharmazeutische Präparate, welche die Verbindungen der allgemeinen Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Darüberhinaus können sie auch zur Behandlung erektiler Dysfunktionen eingesetzt werden. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z. B. 2 bis 4 Teilverabreichungen aufgeteilt.

### Beispiele

### 1a) 4-Hydroximino-3-(pyrid-3-yl)-isoxazol-5-on

Eine Suspension von 57 g 3-(Pyrid-3-yl)-isoxazol-5-on in 50 ml Wasser und 350 ml Eisessig wird bei 15^{o}C mit einer Lösung von 27 g Natriumnitrit in 60 ml Wasser tropfenweise versetzt. Nach 4 Stunden wird der Niederschlag abgesaugt, mit Wasser gewaschen und getrocknet.
Ausbeute: 53 g
Fp.: 192^{o}C (Zers.)

### b) Allgemeine Vorschrift für die Herstellung von 3-(Pyrid-3-yl)-2,3-dioximino-propionamiden

Eine Lösung von 0,05 mol 4-Hydroximino-3-(pyrid-3-yl)-isoxazol-5-on in 50 ml Methanol wird bei Raumtemperatur mit einem Amin HNR₃R₄ (0,055 mol) versetzt und mehrere Stunden bis Tage sich selbst überlassen. Der Niederschlag wird abgesaugt und mit Methanol gewaschen. Fällt kein Niederschlag aus, so wird am Rotationsverdampfer eingeengt und der Rückstand aus einem geeigneten LM umkristallisiert oder säulenchromatographisch (Kieselgel) gereinigt. Auf diese Weise werden hergestellt:
1. 3-(Pyrid-3-yl)-2,3-dioximinopropionsäuremethylamid
   Fp.: 153^{o}C (Zers.)
2. 3-(Pyrid-3-yl)-2,3-dioximinopropionsäurehexylamid
   Fp.: 201^{o}C (Zers.)
3. 3-(Pyrid-3-yl)-2,3-dioximinopropionsäurebenzylamid
   Fp.: 140^{o}C (Zers.)
4. 3-(Pyrid-3-yl)-2,3-dioximinopropionsäurepropylamid
   Fp.: 184^{o}C (Zers.)
5. 3-(Pyrid-3-yl)-2,3-dioximinopropionsäure-(2-diethylaminoethyl)-amid
   Fp.: 174^{o}C (Zers.)

### c) Allgemeine Vorschrift für die Herstellung von Pyrid-3-yl-1,2, 5-oxadiazol-carbonsäureamid-N-oxiden

Eine Mischung von 0,02 mol einer Verbindung gemäß 1b) und 20 ml Wasser wird mit 2 N Natronlauge versetzt, bis alles in Lösung gegangen ist. Nach Abkühlen im Eisbad wird eine 14 %ige Chlorbleichlauge (0,022 mol) zugetropft und der Niederschlag nach 15 Minuten abgesaugt. Falls sich ein Öl abscheidet, wird das Produkt mit Essigester oder Methylenchlorid extrahiert. Der Feststoff oder das erhaltene Öl wird säulenchromatographisch (Kieselgel, Laufmittel: Cyclohexan/Essigester) aufgearbeitet, wobei zwei isomere Produkte erhalten werden, die sich durch Auflösen in Essigester/Ethergemischen mit Salzsäure in die Hydrochloride überführen lassen. Auf diese Weise werden hergestellt:
1. 3-(Pyrid-3-yl)-1,2,5-oxadiazol-4-carbonsäuremethyl amid-2-oxid
   Fp.: 189 - 191^{o}C
2. 4-(Pyrid-3-yl)-1,2,5-oxadiazol-3-carbonsäuremethylamid-2-oxid
   Fp.: 151 - 153^{o}C
3. 4-(Pyrid-3-yl)-1,2,5-oxadiazol-3-carbonsäurehexylamid-2-oxid
   Fp.: Öl
4. 4-(Pyrid-3-yl)-1,2,5-oxadiazol-3-carbonsäurehexylamid-2-oxid-hydrochlorid
   Fp.: 143^{o}C (Zers.)
5. 4-(Pyrid-3-yl)-1,2,5-oxadiazol-3-carbonsäurebenzylamid-2-oxid; Fp.: 85 - 87^{o}C
6. 3-(Pyrid-3-yl)-1,2,5-oxadiazol-4-carbonsäurebenzylamid-2-oxid; Fp.: 135 - 137^{o}C
7. 4-(Pyrid-3-yl)-1,2,5-oxadiazol-3-carbonsäurepropylamid-2-oxid
   Fp.: 62 - 63^{o}C
8. 4-(Pyrid-3-yl)-1,2,5-oxadiazol-3-carbonsäure-(2-diethylaminoethyl)-amid-2-oxid
   Fp: Öl
9. 4-(Pyrid-3-yl)-1,2,5-oxadiazol-3-carbonsäure-(2-methoxyethyl)-amid-2-oxid-hydrochlorid
   Fp.: 145 - 148^{o}C
10. 3-(Pyrid-3-yl)-1,2,5-oxadiazol-4-carbonsäure-(2-methoxyethyl)-amid-2-oxid
   Fp.: 84 - 86^{o}C
11. 3-(Pyrid-3-yl)-1,2,5-oxadiazol-4-carbonsäure-(2-diethylaminoethyl)-amid-2-oxid
   Öl
12. 3-(Pyrid-3-yl)-1,2,5-oxadiazol-4-carbonsäure-(2-hydroxyethyl)-amid-2-oxid
   Fp.: 178 - 179^{o}C
13. 4-(Pyrid-3-yl)-1,2,5-oxadiazol-3-carbonsäure-(2-hydroxyethyl)-amid-2-oxid
   Fp.: 147 - 149^{o}C
14. 3-(Pyrid-3-yl)-1,2,5-oxadiazol-4-carbonsäure-(3-imidazol-1-yl-propyl)-amid-2-oxid
   Fp.: 104 - 106^{o}C
15. 4-(Pyrid-3-yl)-1,2,5-oxadiazol-3-carbonsäure-(3-imidazol-1-yl-propyl)-amid-2-oxid
   Fp.: 88 - 89^{o}C
16. 4-(Pyrid-3-yl)-1,2,5-oxadiazol-3-carbonsäure-(aminocarbonylmethyl)-amid-2-oxid
   Fp.: 197 - 198^{o}C
17. 4-(Pyrid-3-yl)-1,2,5-oxadiazol-3-carbonsäure-(2-di-isopropylamino-ethyl)-amid-2-oxid-hydrochlorid
   Fp.: 191^{o}C (Zers.)
18. 3-(Pyrid-3-yl)-1,2,5-oxadiazol-4-carbonsäure-(2-di-isopropylamino-ethyl)-amid-2-oxid-hydrochlorid
   Fp.: 191^{o}C (Zers.)
19. 4-(Pyrid-3-yl)-1,2,5-oxadiazol-3-pyrrolidin-1-yl-carbonyl-2-oxid
   Fp.: 112 - 114^{o}C
20. 4-(Pyrid-3-yl)-1,2,5-oxadiazol-3-carbonsäure-(2-diethylamino-ethyl)-amid-2-oxid-hydrofumarat
   Fp.: 122 - 123^{o}C
21. 4-(Pyrid-3-yl)-1,2,5-oxadiazol-3-carbonsäure-(3-dimethylamino-propyl)-amid-2-oxid
   Öl
22. 4-(Pyrid-2-yl)-1,2,5-oxadiazol-3-carbonsäure-(2-diethylamino-ethyl)-amid-2-oxid-hydrofumarat
   Fp.: 148 - 151^{o}C (Zers.)
23. 3-(Pyrid-3-yl)-1,2,5-oxadiazol-4-carbonsäure-(3-dimethylaminopropyl)-amid-2-oxid
   Öl
24. 3-(Pyrid-4-yl)-1,2,5-oxadiazol-4-carbonsäuremethylamid
   Fp.: 195 - 196^{o}C
25. 4-(Pyrid-4-yl)-1,2,5-oxadiazol-3-carbonsäuremethylamid
   Fp.: 155 - 156^{o}C
26. 4-(Pyrid-4-yl)-1,2,5-oxadiazol-3-carbonsäure-(3-imidazol-1-yl-propyl)-amid-2-oxid-hydrofumarat
   Fp.: 151^{o}C (Zers.)
27. 3-(Pyrid-4-yl)-1,2,5-oxadiazol-4-carbonsäure-(3-imidazol-1-yl-propyl)-amid-2-oxid-hydrofumarat
   Fp.: 153^{o}C (Zers.)
28. 3-(Pyrid-3-yl)-1,2,5-oxadiazol-4-carbonsäureamid-2-oxid Fp.: 193^{o}C (Zers.).

Die pharmakologische Wirkung der Verbindungen der allgemeinen Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l CaCl₂ löst dann eine Kontraktion aus.
Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= IC₅₀, mol/l).

Folgende Werte wurden erhalten:

| Verbindung | IC₅₀ (mol/l) |
|---|---|
| 2 | 7·10⁻⁷ |
| 3 | 3·10⁻⁷ |
| 7 | 2·10⁻⁷ |
| 8 | 1·10⁻⁷ |
| 10 | 1·10⁻⁶ |
| 15 | 4·10⁻⁷ |
| 16 | 2·10⁻⁶ |
| 17 | 9·10⁻⁸ |
| 18 | 5·10⁻⁷ |
| 23 | 4·10⁻⁶ |
| 24 | 2·10⁻⁶ |
| 25 | 1·10⁻⁶ |
| Molsidomin (Vergleich) | 3·10⁻⁴ |
| Isosorbit-5-mononitrat (Vergleich) | > 1·10⁻⁴ |

## Patentansprüche

1. Pyridyl-1,2,5-oxadiazol-carbonamid-2-oxide der allgemeinen Formel I 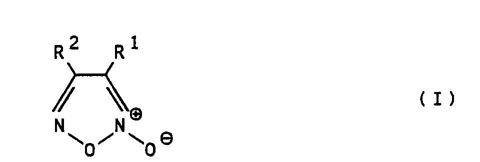 worin einer der Reste R¹ und R² für Pyridyl und der andere für 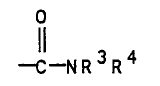 steht,
wobei
R³ und R⁴ unabhängig voneinander Wasserstoff, (C₁-C₆)-Alkyl, (C₅-C₇)-Cycloalkyl, -(CH₂)ₙ-NR⁵R⁶, -(CH₂)ₙ-OR⁵, -(CH₂)ₘ-COOR⁵, -CH(Alk)-COOR⁵, -(CH₂)ₘ-CONR⁵R⁶, -CH(Alk)-CONR⁵R⁶, 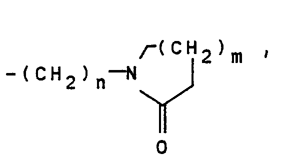 -(CH₂)ₘ-Aryl oder -(CH₂)ₘ-Heteroaryl bedeuten oder R³ und R⁴ zusammen mit dem diese bindenden Stickstoffatom einen Heterocyclus bilden;
R⁵ und R⁶ unabhängig voneinander Wasserstoff, (C₁-C₆)-Alkyl, (C₅-C₇)-Cycloalkyl, Benzyl, Phenethyl bedeuten;
Alk (C₁-C₆)-Alkyl bedeutet, sowie
n für 2, 3 oder 4 und
m für 0, 1, 2 oder 3 stehen,
sowie deren pharmakologisch annehmbare Säureadditionsverbindungen.

2. Pyridyl-1,2,5-oxadiazol-carbonamid-2-oxide gemäß Anspruch 1, dadurch gekennzeichnet, daß R³ und R⁴ unabhängig voneinander Wasserstoff oder (C₁-C₆)-Alkyl bedeuten.

3. Pyridyl-1,2,5-oxadiazol-carbonamid-2-oxide gemäß Anspruch 1 und/oder 2, dadurch gekennzeichnet, daß R³ Methyl und R⁴ Wasserstoff bedeuten.

4. Pyridyl-1,2,5-oxadiazol-carbonamid-2-oxide gemäß Anspruch 1, dadurch gekennzeichnet, daß R³ -(CH₂)ₙN((C₁-C₆)-Alkyl)₂ und R⁴ Wasserstoff bedeuten.

5. Pyridyl-1,2,5-oxadiazol-carbonamid-2-oxide gemäß einem oder mehreren der Ansprüche 1 bis 4, dadurch gekennzeichnet, daß der für R¹ oder R² stehende Pyridylrest Pyrid-3-yl ist.

6. Verfahren zur Herstellung von Pyridyl-1,2,5-oxadiazol-carbonamid-2-oxiden der allgemeinen Formel I gemäß einem oder mehreren der Ansprüche 1 bis 5, dadurch gekennzeichnet, daß eine Verbindung der allgemeinen Formel II 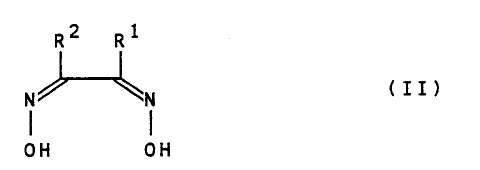 worin R¹ und R² wie in Anspruch 1 angegeben definiert sind, oxidiert wird.

7. Verfahren zur Herstellung von Pyridyl-1,2,5-oxadiazolcarbonamid-2-oxiden der allgemeinen Formel I gemäß einem oder mehreren der Ansprüche 1 bis 5, dadurch gekennzeichnet, daß eine Verbindung der allgemeinen Formel IV 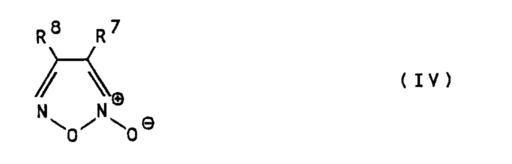 worin einer der Reste R⁷ und R⁸ für Pyridyl und der worin einer der Reste R⁷ und R⁸ für Pyridyl und der andere für eine reaktive Säuregruppe steht, mit einem Amin HNR³R⁴, worin R³ und R⁴ wie in Anspruch 1 angegeben definiert sind, umgesetzt wird.

8. Verwendung von Pyridyl-1,2,5-oxadiazol-carbonamid-2-oxid der allgemeinen Formel I gemäß einem der Ansprüche 1 bis 5 zur Bekämpfung und Vorbeugung von Erkrankungen des kardiovaskulären Systems.

9. Verwendung von Pyridyl-1,2,5-oxadiazol-carbonamid-2-oxid der allgemeinen Formel I gemäß einem der Ansprüche 1 bis 5 zur Behandlung erektiler Dysfunktionen.

10. Pharmazeutisches Präparat, dadurch gekennzeichnet, daß es ein Pyridyl-1,2,5-oxadiazol-carbonamid-2-oxid der allgemeinen Formel I gemäß einem der Ansprüche 1 bis 5, oder ein pharmakologisch annehmbares Säureadditionssalz davon als Wirkstoff zusammen mit pharmazeutisch annehmbaren Träger- und Zusatzstoffen und gegebenenfalls noch ein oder mehrere andere pharmakologische Wirkstoffe enthält.
